(19) Europäisches Patentamt
European Patent Office
Office européen des brevets

(11) Publication number: **0 035 412**
**A1**

# (12) EUROPEAN PATENT APPLICATION

(21) Application number: **81300916.4**

(22) Date of filing: **05.03.81**

(51) Int. Cl.[3]: **C 07 D 403/12**, A 61 K 31/41
// (C07D403/12, 257/06, 209/14)

(30) Priority: **05.03.80 GB 8007546**

(43) Date of publication of application: **09.09.81**
**Bulletin 81/36**

(84) Designated Contracting States: **AT BE CH DE FR GB IT LI NL SE**

(71) Applicant: **GLAXO GROUP LIMITED, Clarges House 6-12 Clarges Street, London W1Y 8DH (GB)**

(72) Inventor: **Guerrato, Alfredo, Via Panisacco 4, I-36070 Trissino (IT)**
Inventor: **Tamburini, Bruno, Via Scuderlando 126, I-37100 Verona (IT)**
Inventor: **Valsecchi, Bruno, Via Zecchinato 4, I-37100 Verona (IT)**

(74) Representative: **Kyle, Diana et al, ELKINGTON AND FIFE High Holborn House 52/54 High Holborn, London WCIV 6SH (GB)**

(54) **Amidotetrazole compounds and processes for their preparation.**

(57) Compounds are disclosed of general formula (I):

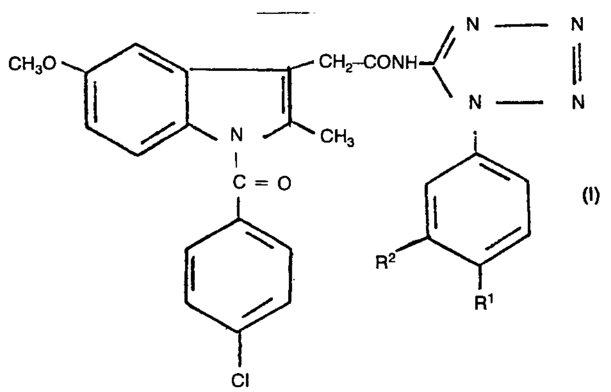

(I)

The compounds may in general be prepared by a condensation reaction or where $R^1$ represents an amino group by reduction of the corresponding compound where $R^1$ represents a nitro group.

The compounds are described as having anti-inflammatory and analgesic activity as well as good therapeutic indices.

**EP 0 035 412 A1**

0035412

## TITLE

# Amidotetrazole Compounds and Processes for their Preparation.

This invention relates to amidotetrazole compounds, to processes for their preparation and to pharmaceutical compositions containing them.

More particularly the invention relates to certain 1-phenyl-5-amidotetrazoles and processes for their preparation.

The present invention provides a compound of general formula (I):

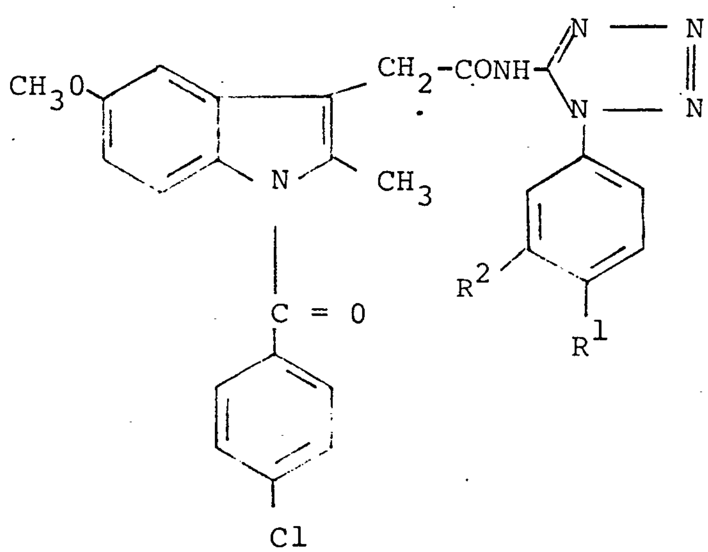

(I)

wherein $R^1$ is a hydrogen atom or a nitro or amino group and $R^2$ is a hydrogen atom or $R^1$ and $R^2$ are both chlorine atoms.

A particularly preferred compound according to the invention is 1-phenyl-5-[1-(4-chlorobenzoyl)-5-methoxy-2-methylindol-3-ylacetamido]tetrazole.

Compounds of general formula (I) may be prepared by

condensing an activated derivative of general formula (II):

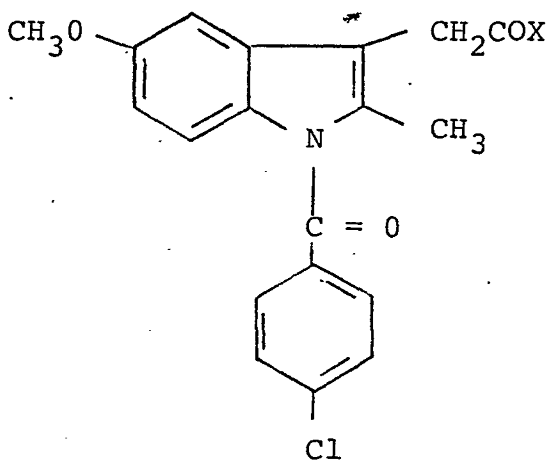

(II)

wherein X is a leaving group

with a 1-phenyl-5-aminotetrazole of general formula (III):

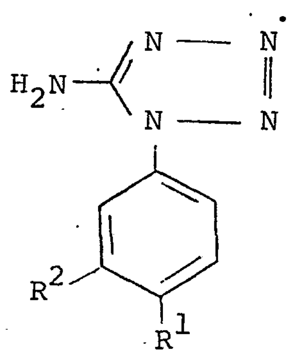

(III)

where $R^1$ and $R^2$ are as defined for formula (I) except that $R^1$ is not an amino group, to give the desired compound of general formula (I) where $R^1$ and $R^2$ are as defined for formula (III).

The compound of formula (I) where $R^1$ is an amino group may be prepared by reduction of the compound of formula (I) where $R^1$ is a nitro group. The reduction may be performed, for example, by hydrogen in the presence of a catalyst, such as platinum, in a suitable solvent, such as

a mixture of tetrahydrofuran and anhydrous ethanol.

The activated derivative of general formula (II) may be, for example, an acid halide such as the acid chloride, an ester such as the p-nitrophenyl or pentachlorophenyl ester, an acid anhydride or a product of the reaction of the free acid of formula (IV):

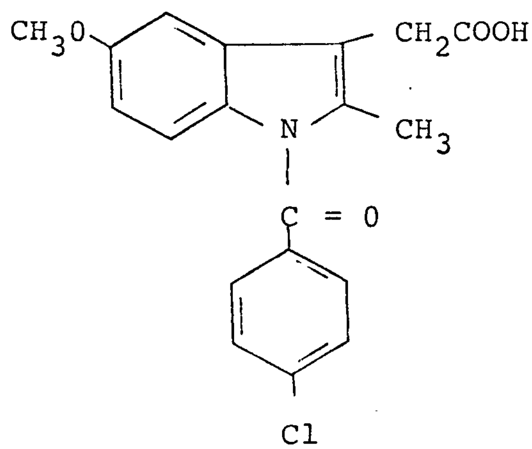

(IV)

with a coupling agent such as carbonyl diimidazole or dicyclohexylcarbodiimide.

The condensation reaction of the derivatives of general formula (II) with the 1-phenyl-5-aminotetrazoles of formula (III) is generally performed in a solvent, optionally at an elevated temperature. The precise reaction conditions however, depend on the nature of the activated derivative used. Thus, for example, when an acid halide is used the reaction may be performed in a solvent such as tetrahydrofuran in the presence of an organic base such as pyridine or triethylamine at a temperature such as the reflux temperature.

The activated derivatives of general formula (II) may be prepared from the acid of formula (IV) by known procedures.

For example, the acid chloride of formula (II) may

be prepared by reaction of the acid of formula (IV) with thionyl chloride,phosphorus pentachloride or oxalyl chloride preferably in a solvent such as cyclohexane at the reflux temperature. An ester of formula (II) may be prepared, for example, by reaction of the acid of formula (IV) with an alcohol preferably in the presence of an acid catalyst such as a mineral acid, for example hydrochloric acid. An anhydride of formula (II) may be prepared, for example, by reaction of the acid of formula (IV) with an acid anhydride such as trifluoroacetic anhydride, an acid chloride such as acetyl chloride or an alkyl or aralkyl haloformate such as ethyl or benzyl chloroformate.

The acid of formula (IV) is a known compound which may be prepared, for example, by the method described in British Patent Specification No. 997638. The aminotetrazoles of formula (III) are also either known compounds or may be prepared by conventional methods from known starting materials for example, by the methods described in British Patent Specification No. 1,208,116 or by Garbretch et al in Journal of Organic Chemistry, Volume 18, page 1020 (1953).

We have found that the compounds of general formula (I) exhibit both anti-inflammatory activity and analgesic activity in the rat. Moreover, the compounds have good therapeutic indices, the therapeutic index of 1-phenyl-5-[1-(4-chlorobenzoyl)-5-methoxy-2-methylindol-3-ylacetamido]tetrazole being particularly advantageous. The anti-inflammatory activity was determined by the Freund adjuvant arthritis test (Pearson, C.M. Proc. Soc. Exp. Biol. Med 91, 95 1956) and analgesic activity was determined by applying an increasing pressure to the inflamed paw (Randall, L.O. Selitto, J.J., Arch. Int. Pharmacodyn, 111, 409 1957) and recording pain threshold.

These tests indicate the potential use of the compounds of general formula (I) for the treatment of inflammatory

diseases, in particular, chronic inflammatory diseases such as arthritis.

Thus, the present invention further provides a method for the treatment of a patient suffering from an inflammatory disease which comprises administering to the patient an effective amount of a compound of general formula (I).

The invention also provides a pharmaceutical composition comprising a compound of general formula (I) together with a physiologically acceptable carrier or excipient.

The compounds may be formulated in conventional manner for administration by any convenient route for example, for oral, rectal, or parenteral administration.

For oral administration, the pharmaceutical composition may take the form of, for example, tablets, capsules, powders, syrups or suspensions prepared by conventional means with physiologically acceptable excipients.

The compounds of general formula (I) may be formulated for rectal administration for example, in the form of suppositories using a conventional suppository excipient. The compounds may also be formulated for parenteral administration in dry form for reconstitution before use, or as a sterile suspension.

A proposed daily dose for administration to man is 50 mg to 2500 mg total daily dose which may be conveniently administered in one to four doses per day.

The invention is further illustrated by the following Examples. The abbreviation THF is used to represent tetrahydrofuran.

EXAMPLE 1

1-Phenyl-5-[1-(4-chlorobenzoyl)-5-methoxy-2-methylindol-3-ylacetamido]tetrazole

(a) 1-(4-chlorobenzoyl)-5-methoxy-2-methylindol-3-ylacetyl chloride

A mixture of 1-(4-chlorobenzoyl)-5-methoxy-2-methylindol-3-yl acetic acid (16.0 g) and thionyl chloride (7.5 g) in cyclohexane (100 ml) was refluxed for 2 hours until a solid precipitated. The mixture was cooled and the precipitate was collected by filtration and washed with petroleum ether (b.p. 60-80°C). The washed precipitate was dried (40°C) to yield the title compound (16.1 g) m.p. 125-127°C.

(b) 1-Phenyl-5-[1-(4-chlorobenzoyl)-5-methoxy-2-methylindol-3-ylacetamido]tetrazole

The chloride of part (a) (19.0 g) in THF (150 ml) was added slowly with stirring to a solution of 1-phenyl-5-aminotetrazole (11.0 g) and pyridine (5 ml) in THF (100 ml). The mixture was refluxed for 5 hours then cooled and filtered. The filtrate was collected and the solvent distilled off under reduced pressure to leave a brown oily residue. The residue was added to methanol to precipitate a solid which was collected by filtration and washed with hot methanol to yield the title compound (15.5 g) m.p. 183-185°C I.R. 3200 (W), 1715 (m), 1680 (s,sh), 755 (s) $cm^{-1}$.

EXAMPLE 2

1-(3,4-Dichlorophenyl)-5-[1-(4-chlorobenzoyl)-5-methoxy-2-methylindol-3-ylacetamido]tetrazole

A solution of the product of Example 1(a) (13.1 g) in THF (100 ml) was added dropwise to a mixture of 1-(3,4-dichlorophenyl)-5-aminotetrazole (10 g), triethylamine (5 ml) and THF (200 ml). The mixture was refluxed for 2h, cooled, filtered and the solvent was removed under reduced pressure to give a residue. A mixture of cyclohexane/

ethyl acetate/acetone (4:4:2) was added, the mixture was filtered and the solvent removed under reduced pressure to give a yellow gum which crystallised from ethanol to yield the title compound (2.15 g) m.p. 173-175°C.

Found: C% 54.74 (Calc. 54.80); H% 3.25 (3.36); N% 14.93 (14.74); Cl% 18.44 (18.66).

EXAMPLE 3

1-(4-Nitrophenyl)-5-[1-(4-chlorobenzoyl)-5-methoxy-2-methylindol-3-ylacetamido] tetrazole

A solution of the product of Example 1(a) (95 g) in THF (500 ml) was added dropwise to a mixture of 1-(4-nitrophenyl)-5-aminotetrazole (62 g) triethylamine (30 ml) and THF (900 ml), refluxed for 13h, cooled and filtered. The solvent was removed under reduced pressure to give a solid gum which was dissolved in ethyl acetate (1000 ml) and washed with 10% hydrochloric acid and water. By partial evaporation of the solvent a precipitate was obtained which was filtered and crystallised from ethanol to yield the title compound (47 g) m.p. 198-200°C.

Found: C% 53.42 (Calc. 57.19); H% 3.34 (3.69); N% 17.12 (17.96); Cl% 6.26 (6.49).

EXAMPLE 4

1-(4-Aminophenyl)-5-[1-(4-chlorobenzoyl)-5-methoxy-2-methylindol-3-ylacetamido]tetrazole

The product of Example 3 (18 g) was dissolved in a mixture of THF and anhydrous ethanol (4:1) and reduced by catalytic hydrogenation ($PtO_2$; 3 atm. of hydrogen). The catalyst was filtered off and the solvent was removed under reduced pressure to obtain a solid. The solid was treated with hot methanol, filtered, and the title compound (2 g) m.p. 185-187°C obtained by repeated chromatography on silica gel using a cyclohexane/ethyl acetate/acetone (4:4:2) mixture as eluent.

Found: C% 60.49 (Calc. 60.52); H% 4.27 (4.29); N% 19.14 (19); Cl% 7.02 (6.87).

EXAMPLE 5

The following are examples of different types of pharmaceutical compositions according to the invention.

Capsules

| | mg/capsule |
|---|---|
| Active ingredient | 100.0 |
| Microcrystalline cellulose | 97.0 |
| Magnesium stearate | 3.0 |
| Fill Weight | 200.0 |

Tablets

| Film Coated | mg/tablet |
|---|---|
| Core | |
| Active ingredient | 100.0 |
| Microcrystalline cellulose | 97.0 |
| Magnesium stearate | 3.0 |
| Film coating | |
| Hydroxypropylmethylcellulose | 4.5 |
| Titanium dioxide | 1.2 |
| Weight | 205.7 |

Suppositories

| | mg/suppository |
|---|---|
| Active ingredient | 200.0 |
| Suppository base* | 2500.0 |
| Weight | 2700.0 |

*Any conventional base may be used.

The anti-inflammatory activity of the compounds was determined in rats with adjuvant arthritis (Pearson, C.M. Prod. Soc. Exp. Biol. Med., 91, 95, 1956) induced by intradermal injection into the tail of 0.1 ml of a fine suspension of *Mycobacterium butyricum* in liquid paraffin (6 $mg.ml^{-1}$).

After 14 days, animals were chosen on the basis of their arthritic score and test compounds, suspended in gum acacia, were then orally administered once a day for 14 days. At the end of this period, animals were scored again and percentage inhibition versus first score was calculated. Comparisons with controls were performed by the Dunnett test. The following results were obtained for the compounds of the Examples:

| Example No. | Oral Dose. $mg.kg^{-1}$ | % Inhibition |
|---|---|---|
| 1 | 6.25 | 59 |
| 2 | 50.0 | 40.3 |
| 3 | 10.0 | 47.7 |
| 4 | 50.0 | 44.8 |

Toxicity

The compounds are in general non-toxic at therapeutically active doses. For example, the compound of Example 1 has an $LD_{50}$ value in the rat in excess of 3000 $mg.kg^{-1}$ orally.

CLAIMS:

1. A compound of general formula (I):

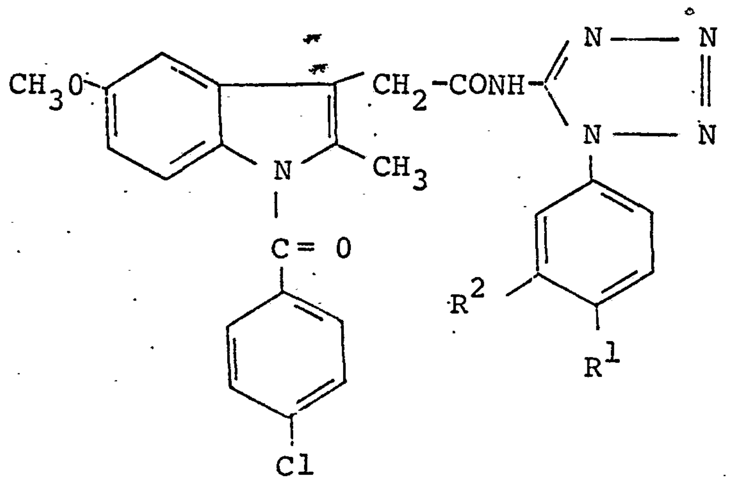

(I)

wherein $R^1$ is a hydrogen atom or a nitro or amino group and $R^2$ is a hydrogen atom or $R^1$ and $R^2$ both represent chlorine atoms.

2. 1-Phenyl-5-[1-(4-chlorobenzoyl)-5-methoxy-2-methylindol-3-ylacetamido] tetrazole.

3. A method of preparing a compound of general formula (I), wherein $R^1$ and $R^2$ are as defined in claim 1 except that $R^1$ is not an amino group, which comprises condensing an activated derivative of general formula (II):

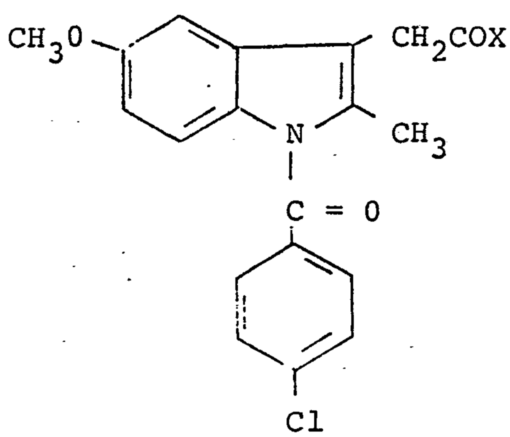

(II)

where X is a leaving group
with a 1-phenyl-5-aminotetrazole of general formula (III):

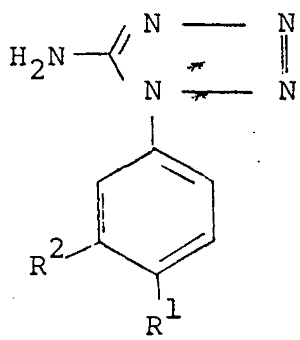

(III)

where $R^1$ is a hydrogen atom or a nitro group and $R^2$ is a hydrogen atom or $R^1$ and $R^2$ both represent chlorine atoms
and recovering the desired compound of general formula (I).

4. A method of preparing a compound of formula (I) as defined in claim 1 wherein $R^1$ is an amino group which comprises reducing a compound of formula (I) where $R^1$ is a nitro group and recovering the desired compound of formula (I), where $R^1$ is an amino group.

5. A pharmaceutical composition which comprises, as active ingredient at least one compound of general formula (I) as defined in claim 1 together with a physiologically acceptable carrier or excipient therefor.

6. A pharmaceutical composition which comprises as active ingredient 1-phenyl-5-[1-(4-chlorobenzoyl)-5-methoxy-2-methylindol-3-ylacetamido] tetrazole.

7. A method according to claim 3, substantially as herein described with reference to any of the specific Examples 1 to 3.

8. A method according to claim 4, substantially as herein described with reference to Example 4.

9. A pharmaceutical composition according to claim 5 substantially as herein described with reference to Example 5.

10. Use of a compound of general formula (I) as defined in claim 1 as an anti-inflammatory and/or analgesic agent.

European Patent Office

**PARTIAL EUROPEAN SEARCH REPORT**
which under Rule 45 of the European Patent Convention shall be considered, for the purposes of subsequent proceedings, as the European search report

Application number
0035412
EP 81300916.4

## DOCUMENTS CONSIDERED TO BE RELEVANT

| Category | Citation of document with indication, where appropriate, of relevant passages | Relevant to claim |
| --- | --- | --- |
| | GB - A - 1 343 827 (SCIENCE UNION)<br>+ Totality +<br>-- | 1,3 |
| | US - A - 3 932 416 (BAYS)<br>+ Abstract +<br>---- | 1 |

CLASSIFICATION OF THE APPLICATION (Int. Cl.³)

C 07 D 403/12
A 61 K 31/41//
(C 07 D 403/12
C 07 D 257/06
C 07 D 209/14)

TECHNICAL FIELDS SEARCHED (Int. Cl.³)

C 07 D 403/00
C 07 D 257/00
C 07 D 209/00
A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-9
Claims searched incompletely: -
Claims not searched: 10
Reason for the limitation of the search:

Method for treatment of the human or animal body by therapy (Article 52(4) EPC)

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| Place of search | Date of completion of the search | Examiner |
| --- | --- | --- |
| VIENNA | 07-05-1981 | HAMMER |

EPO Form 1505.1 06.78